# EUROPEAN PATENT APPLICATION

(11) **EP 4 725 524 A1**
(43) Date of publication of application: **15.04.2026**
(21) Application number: 24306646.1
(22) Date of filing: 08.10.2024
(51) Int. Cl.: A61M 5/20, A61M 5/24, A61M 5/32

(54) **OPTICAL SYSTEM, AUTO-INJECTOR COMPRISING SUCH AN OPTICAL SYSTEM AND METHOD FOR DETECTING A POSITION OF A NEEDLE COVER WITH SUCH AN OPTICAL SYSTEM**

(71) Applicant: NEMERA LA VERPILLIERE, 38290 La Verpillière (FR)
(72) Inventor: DELVALAC, Sébastien, 69007 LYON (FR); CASSAGNE, Loick, 38080 SAINT-ALBAN-DE-ROCHE (FR)
(74) Representative: Lavoix

(57) **Abstract**

This optical system (80) detects a position of a needle cover (18) in an auto-injector, said needle cover being able to translate between a retracted position and an extended position. The optical system comprises at least one light source (824), at least one light detector (826B); and a barrier (846) able to move in front of the light detector. A movement of the needle cover (18) along a longitudinal axis (X2) causes an axial displacement of the barrier (846) and changes a light intensity measured by the light detector (826B). The optical system (80) is configured to detect an axially retracted position of the needle cover (18) due to a variation of the light intensity measured by the light detector (826B).

## Description

The present invention concerns an optical system for detecting a position of a needle cover in an auto-injector. The present invention also concerns an auto-injector comprising an injection needle, a needle cover and an optical system for detecting a position of the needle cover. Finally, the invention concerns a method for detecting a position of a needle cover in an auto-injector, thanks to an optical system.

### TECHNICAL FIELD OF INVENTION

The invention belongs to the technical field of auto-injectors used for injecting a medicine in a living body, via a needle. In this technical field, it is known to protect such a needle with a needle cover, in order to avoid injuries to the patient or to the practitioner using an auto-injector. The needle cover usually fully covers the needle, so that the needle tip cannot be accessible to the fingers. When contacting the auto-injector to the site of injection, the needle cover usually retracts so that the needle can enter the injection site. The stroke of the needle cover retraction corresponds therefore to the depth of needle insertion. When removing the auto-injector from the injection site, the needle cover usually extends to cover again the needle. In this context, it is useful to know each of the different positions of the needle cover, in order to assess if it the needle has been properly inserted into, for example, a patient body before the injection.

In order to reduce the amount of waste generated by the manufacturing and use of the auto-injectors, some auto-injectors now comprise a reusable module and a disposable module. These modules are to be assembled together by the patient and/or the practitioner before use. After use only the disposable module is to be discarded, while the reusable module can be used many times. In this context, it is, in addition, useful to assess if the two modules have been correctly assembled.

As indicated above, it is of particular importance to detect a retracted position of the needle cover, as it ensures that the needle was properly inserted into the patient's skin and the injection will result in proper drug delivery.

With this respect, EP3630238B1 discloses an electronic patch injector equipped with a printed circuit board carrying an optical sensor perpendicular to a motion of a pivoting needle shield assembly. This device is not compatible with a needle cover having a translational movement between a retracted position and an extended position.

On the other hand, WO2015112392A1 discloses a system for detecting loading of a cassette in an infusion pump, where a light emitter emits a light beam toward a photosensitive detector. This system is not adapted for the detection of the several positions of a needle cover.

A technical problem is how to provide an accurate confirmation of a proper retraction of the needle cover before injection and therefore proper needle insertion. Hence, a need exists for a device capable of efficiently detecting the position of a needle cover movable in translation, in particular a retracted position of the needle cover.

Another technical problem is how to provide, in addition to the detection of the proper retraction of the needle cover, the detection of the assembly/disassembly of the disposable part to the reusable part, preferably using a unique system.

### SUMMARY OF THE INVENTION

The present invention addresses this issue by providing an optical system capable of efficiently detecting a position of a needle cover mobile in translation.

With this respect, according to a first aspect, the present invention relates to an optical system for detecting a position of a needle cover in an auto-injector, said needle cover being able to translate between a retracted position and an extended position, the optical system comprising
- at least one light source;
- at least one light detector; and
- a barrier able to move in front of the light detector,
wherein
- a movement of the needle cover along a longitudinal axis causes an axial displacement of the barrier and changes a light intensity measured by the light detector, and
- the optical system is configured to detect an axially retracted position of the needle cover due to a variation of the light intensity measured by the light detector.

Thanks to the invention, the optical system is efficient to detect when the needle cover reaches its retracted position, based on the variation of the light intensity measured by the light detector. The use of an auto-injector equipped with such an optical system is easier and safer than the use of known detection devices.

According to advantageous and optional aspects of the invention, such an optical system may incorporate one or several of the following features:
- The barrier and the needle cover are two separate elements coupled with each other.
- The barrier is in one part with the needle cover.
- The optical system comprises at least two light detectors.
- During a movement of the needle cover along the longitudinal axis , output signals of the at least two light detectors are shifted in phase.
- The optical sensor comprises at least two light sources.
- The barrier comprises at least one opening.
- The optical system comprises at least two light detectors
- During a movement of the needle cover along the longitudinal axis, output signals of the at least two light detectors change periodically.

According to a second aspect, the invention relates to an auto-injector for use with a syringe comprising an injection needle, the auto-injector comprising a needle cover for covering the injection needle, the needle cover being mobile in translation along an axial direction, between an extended position and a retracted position, wherein the auto-injector comprises an optical system as previously described.

Such an auto-injector may incorporate one or several of the following optional features:
- The auto-injector comprises a reusable module and an disposable module including the needle cover, wherein mounting of the disposable module on the reusable module causes an axial movement of the barrier.
- The optical system is configured to identify a correct mounting of the disposable module on the reusable module.
- The optical system is configured to detect that the needle cover is in its extended position after being previously in the retracted position.
- The optical system is configured to detect if the disposable module is not mounted on the reusable module.
- The auto-injector comprises a reusable module and a disposable module including the needle cover, whereas
   - the light source and the at least one light detector of the optical system belong to the reusable module,
   - the barrier allows the same amount of light to reach the at least one light detector
      - when the disposable module is mounted on the reusable module and the needle cover is in its retracted position and
      - when the disposable module is not mounted on the reusable module.

According to a third aspect, the invention relates to a method for detecting a position of a needle cover in an auto-injector as mentioned here above, comprising :
a) emitting a light beam from the light source toward the at least one light detector;
b) measuring a light intensity via at least one light detector;
c) providing an output signal corresponding to the light intensity measured by the at least one light detector;
d) comparing the output signal of step c) to at least one known threshold value;
e) determining a state of the at least one light detector, based on the comparison of step d);
f) determining the position of the needle cover.

Advantageously, in step e), an assumption is made that the state of the light detector can take one of a first value, a second value and a third value and that each value corresponds to one of the following four configurations: i) no disposable module is mounted on the reusable module; ii) the disposable module is mounted on the reusable module and the needle cover is in its extended position; iii) the disposable module is mounted on the reusable module and the needle cover moves from its extended position to its retracted position; iv) the disposable module is mounted on the reusable module and the needle cover is in its retracted position, whereas the state is the same for the configuration i) and the configuration iv).

Alternatively, in step e), an assumption is made that the state of the two detectors can take one of a first value, and a minimum value, and that each value corresponds to one of the following four configurations are identified, i) no disposable module is mounted on the reusable module; ii) the disposable module is mounted on the reusable module and the needle cover is in its extended position; iii) the disposable module is mounted on the reusable module and the needle cover moves from its extended position to its retracted position; iv) the disposable module is mounted on the reusable module and the needle cover is in its retracted position. Configuration i) corresponds to a state when the two detectors take the first value, configuration ii) corresponds to a state when at least one detector takes the minimum value and configuration iv) corresponds to a state when the two detectors take the first value, following sequential changes in the state of the two detectors starting from the configuration ii).

According to another optional aspect, the light beam is a beam of an infrared light.

### BRIEF DESCRIPTION OF THE DRAWINGS

The invention will be better understood, based on the following description, given as a non-limiting example and made in reference to the following figures:
- [Fig. 1] Figure 1 is a perspective view of an of an auto-injector according to a first embodiment of the invention, which includes an optical system according to a first embodiment of the invention;
- [Fig.2] Figure 2 is a perspective exploded view of the auto-injector of figure 1 and of a syringe usable with this auto-injector;
- [Fig.3] Figure 3 is a perspective view of some parts of the auto-injector of figures 1 and 2, detail A representing a partial cut view along plane A on the main figure;
- [Fig.4] Figure 4 is side view of the parts of the auto-injector visible on figure 3, plus a puller and a cap, in a first configuration;
- [Fig.5] Figure 5 is side view similar to figure 4, of the parts of the auto-injector visible on figure 3, in a second configuration;
- [Fig.6] Figure 6 is side view of some the parts of the auto-injector, in the second configuration;
- [Fig.7] Figure 7 is side view similar to figure 6, of the same parts of the auto-injector, in a third configuration;
- [Fig.8] Figure 8 is side view similar to figure 6, of the same parts of the auto-injector, in a fourth configuration;
- [Fig 9] Figure 9 is a graph showing output signals of the optical sensors of the auto-injector of figures 1 to 8, as a function of time, depending on the position of a needle cover;
- [Fig 10] Figure 10 schematically shows several positions of a barrier with respect to an optical sensor of the optical system of the first embodiment; ;
- [Fig 11] Figure 11 schematically shows several positions of a barrier with respect to an optical sensor of an optical system of a second embodiment of the invention;
- [Fig 12] Figure 12 shows output signals of the optical sensors, in a way comparable to figure 9, for the second embodiment of the invention;
- [Fig 13] Figure 13 schematically shows several positions of a barrier with respect to an optical sensor of an optical system of a third embodiment of the invention; and
- [Fig 14] Figure 14 shows the output signal of an optical sensor, in a way comparable to figure 9, for the third embodiment of the invention.

### DETAILED DESCRIPTION OF SOME EMBODIMENTS

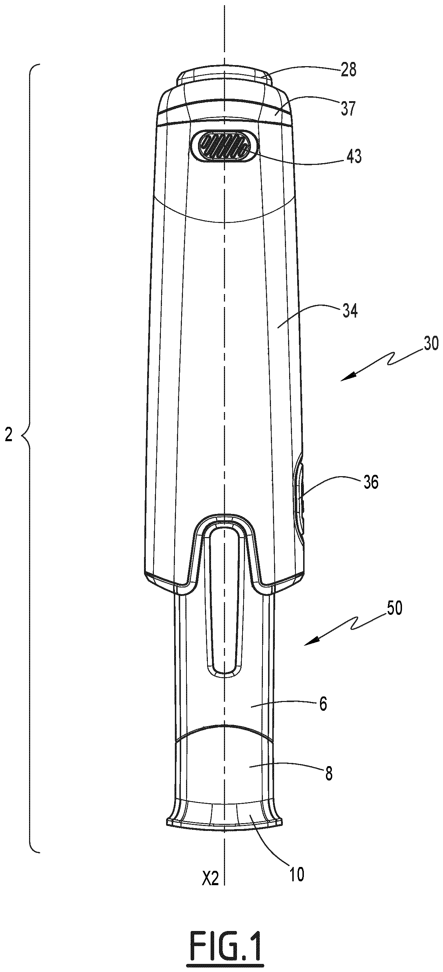

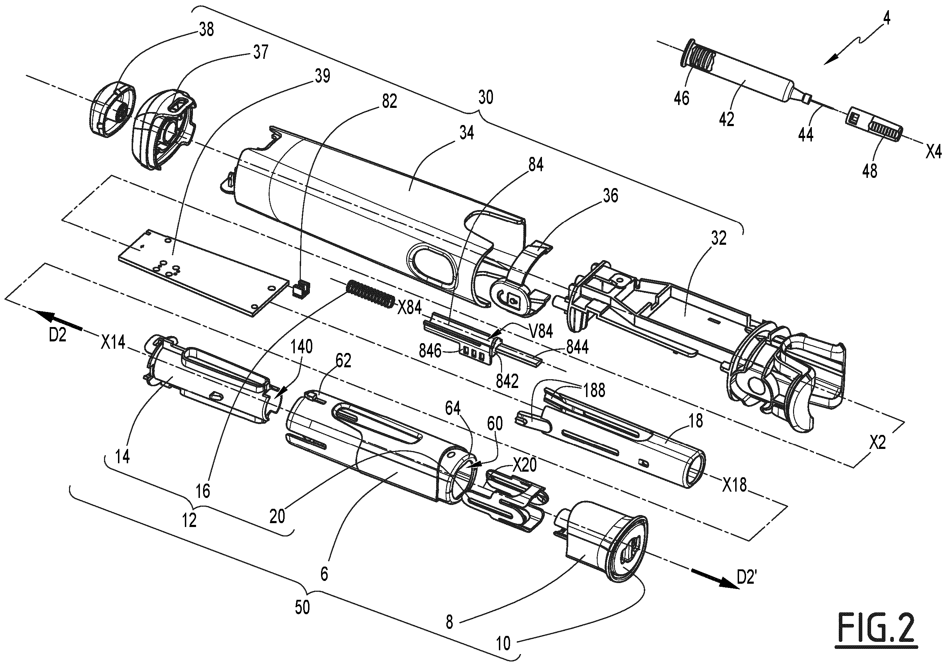

An embodiment of an auto-injector 2 according to the invention is represented on figures 1 to 8 and is configured to accommodate a syringe 4, represented on figure 2 only. The syringe 4 includes a barrel 42, a needle 44, a piston 46 and needle cap 48. On figure 2, the barrel 42 is partly torn off, in order to show the piston 46. Moreover, the needle cap 48 is partly removed, in order to show the needle 44.

The syringe 4 belongs to the auto-injector 2 when it is mounted within the auto-injector 2.

Advantageously, the barrel 42 is transparent.

The auto-injector 2 extends along a longitudinal axis X2.

The auto-injector 2 includes a reusable module 30, which comprises a frame 32, a shell 34, a locking button 36, a light indicator 37, a push button 38, a printed circuit board 39 and a non-represented motor configured for pushing a piston rod exerting a force on the piston 46. A slider control button 43, represented on figure 1 only and mounted on the shell 34, allows controlling the speed of displacement of the piston 46, when it is moved in the barrel 42 by the non-represented motor.

The reusable module 30 also includes an optical system 80, which includes an optical sensor 82 and a shuttle 84 mobile along a direction parallel to the longitudinal axis X2 of the auto-injector 2.

The auto-injector 2 also includes a disposable module 50, which is discarded when the content of the needle 4 has been injected, while the reusable module can be user over a long time.

The disposable module 50 includes a body 6 and a puller 8, said puller 8 further comprising a cap 10, which closes the puller 8 on its side opposite to the body 6. The puller 8 allows extracting the needle cap 48 from the body 6, for example by providing friction interface between these elements when it is necessary to use the needle 44 for injecting a liquid contained in the barrel 42, in particular into a human or animal body.

The syringe 4 extends along a longitudinal axis X4. When the syringe 4 is mounted within the auto-injector 2, the axes X2 and X4 coincide.

62 denotes a first longitudinal end of the body 6, which preferably defines an entry zone for the syringe 4 when the syringe is inserted into the auto-injector 2.

64 denotes a second longitudinal end of the body 6, which is opposite to the first longitudinal end 62 and close to the puller 8 when the puller is mounted on the body 6.

D2 denotes a first longitudinal direction, parallel to the longitudinal axis X2 and oriented from the second end 64 to the first end 62. D2' denotes a second longitudinal direction, opposite to the first longitudinal direction, parallel to the longitudinal axis X2 and oriented from the first end 62 to the second end 64.

The second longitudinal end 64 defines an end opening 60 of the body 6, through which the needle 44 protrudes out of the auto-injector 2, when the liquid contained in the barrel 42 is to be injected within a human or animal body.

The auto-injector 2 is equipped with a safety system 12 for preventing needle-stick injuries when the auto-injector is manipulated, for instance by a patient or a medical practitioner.

The safety system 12 includes a sleeve 14 fixedly assembled within the body 6. In other words, the sleeve 14 is fixed in the body 6, along the longitudinal axis X2 and in any direction perpendicular to this axis.

The fixation of the sleeve 14 within the body 6 may occur in any conventional matter, e.g. by snapping, gluing, welding.

In a non-represented alternative embodiment of the invention, the sleeve 14 may be formed in one piece with the body 6. In other words, the body 6 and the sleeve 14 are monobloc. Such a case should also be regarded as when the sleeve 14 fixed with the body 6, and it is to be understood that the sleeve 14 fixed with the body covers also said monobloc possibility.

The sleeve 14 can be made, for example of, a polymer suitable for injection molding which is preferred way of manufacturing such element. Suitable polymer materials may be composed mainly of mainly of a copolymer of methyl methacrylate, acrylonitrile, butadiene and styrene (MABS), a copolymer of methyl methacrylate butadiene styrene (MMBS), polypropylene (PP), copolymer of acrylonitrile-butadiene-styrene (ABS), polyoxymethylene (POM), polyamide (PA), polybuthylene terephtalate (PBT), or a polycarbonate (PC) or their mixtures.

The sleeve 14 extends along a longitudinal axis X14.

The safety system 12 also includes a biasing element 16.

In a non-limiting example, this biasing element 16 is a spiral, compression spring, as shown on figure 2. The spring is preferably made of metal.

In a variant, this biasing element 16 can be made of a leaf spring, a conical spring, a flexible part of the body 6 or the sleeve 14 or any other means providing biasing force along the axis X2.

The safety system 12 also includes a needle cover 18 configured for surrounding the needle 44, in particular when the needle cap 48 has been removed, in order to hide the needle and prevent needle-stick injuries.

The needle cover 18 is preferably also made of a polymer which can be the same as, or different from, the material of the sleeve 14.

The needle cover 18 is centered on a longitudinal axis X18

The needle cover 18 is equipped with two lugs 188, located diametrically oppositely with respect to its longitudinal axis X18.

In a non-represented variant of the invention, the needle cover 18 includes one leg 188 only.

In assembled configuration of the safety system 12, the needle cover 18 is mounted around the sleeve 14.

Advantageously, in this assembled configuration, the longitudinal axes X14 and X18 coincide.

The safety system 12 also includes a locker 20, which is an annular element centered on a longitudinal axis X20.

In assembled configuration of the safety system 12, the locker 20 is mounted around the needle cover 18. Advantageously, in this assembled configuration, axes X14, X18 and X20 coincide.

Advantageously, each one of parts 14, 18 and 20 is made in one piece, in other words monobloc.

Parts 14, 16, 18 and 20 together belong to the safety system 12. Within the safety system 12, parts 14, 18 and 20 belong to the disposable module 50, whereas the spring 16 belongs to the reusable module 30.

Advantageously, the safety system 12 is built according to the teachings of the European patent application filed on the same day as the present application, in the name of the same Applicant, and concerning a safety system for an injection device and an injection device comprising such a safety system.

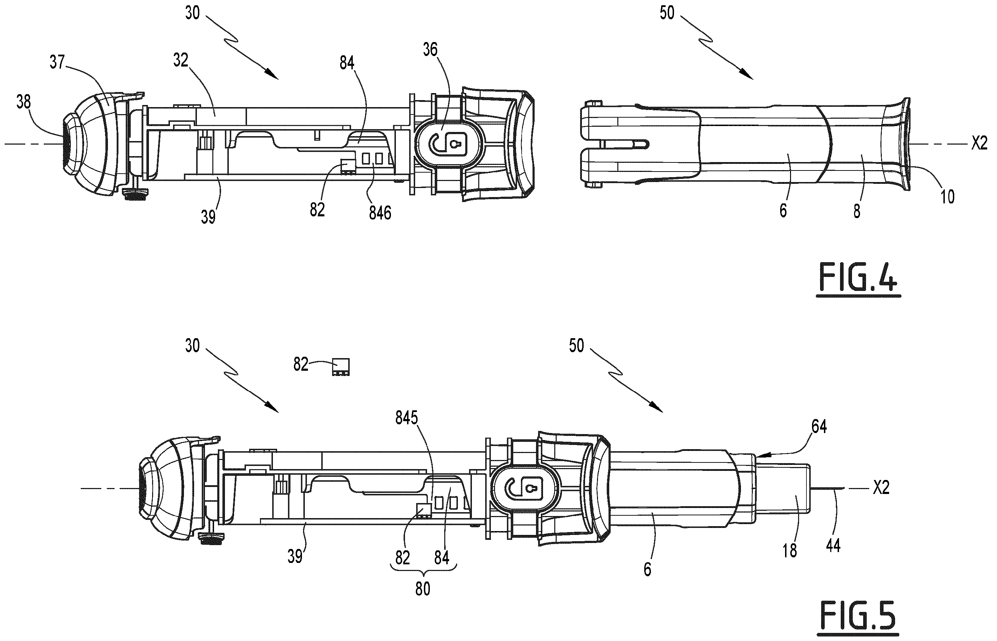

During the lifetime of the reusable module 30, it can be used many times with subsequent disposable modules 50. Each time, preferably before and after use of the auto-injector 2, the disposable module 50 can be separated from the reusable module 30, as represented on figure 4, and the needle cover 18 can take several positions along the longitudinal axis X2, with respect to the reusable module 30, as represented respectively on figures 5 to 8.

The optical system 80 allows determining if the disposable module 50 is correctly mounted on the reusable module 30 and, when such is the case, to know the actual position of the needle cover 18 along the longitudinal axis X2.

In the presented embodiment the optical sensor 82 includes a U shaped body 822 fixedly mounted on the printed circuit board 39. The optical sensor 82 also includes a light source 824 and at least one light detector. In the presented embodiment the optical sensor 82 comprises a first light detector 826A and a second light detector 826B. Along the longitudinal axis X2, the first light detector 826A is located before the second detector in the direction D2' and after the second detector in the direction D2. The light source 824 and the light detectors 826A and 826B respectively mounted on two legs of the body 822, on either side of a median plane P82 of the optical sensor 82.

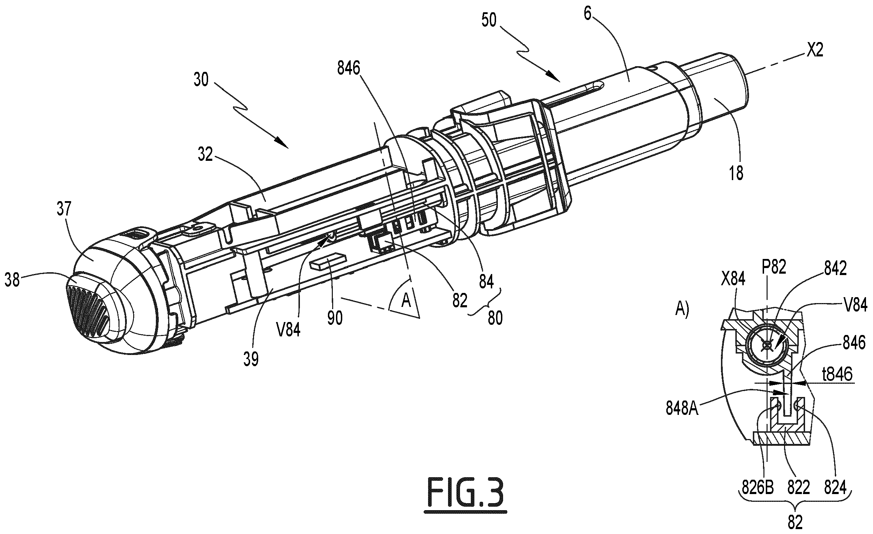

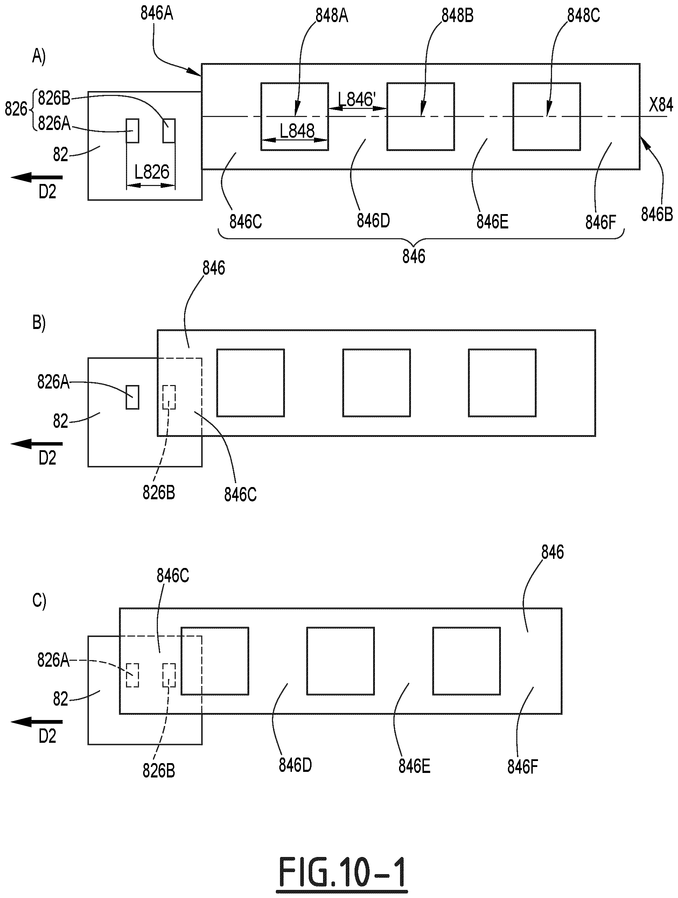

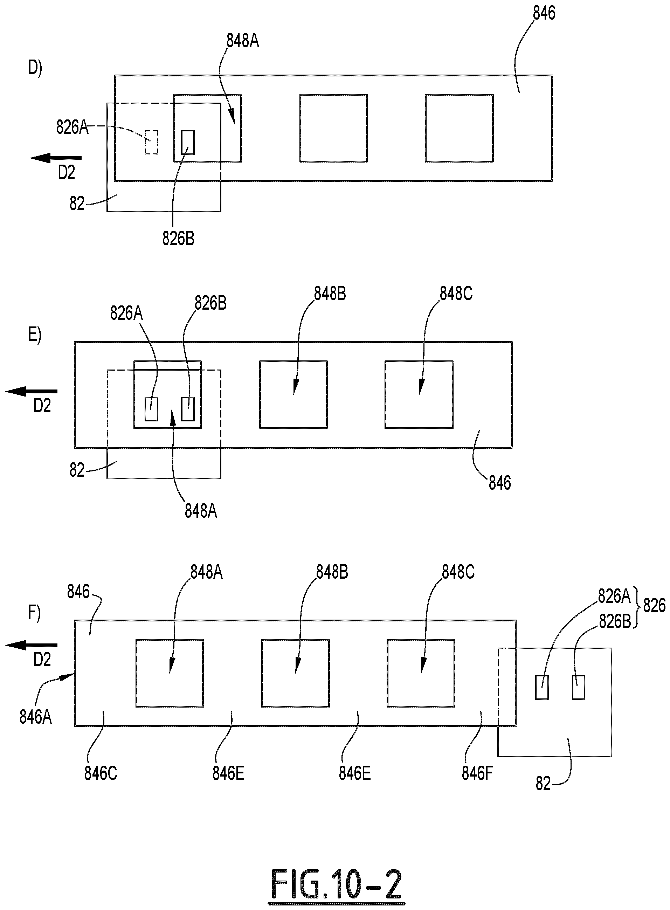

Only one light detector 826B is visible on figure 3. Both light detectors 826A and 826B are represented on figure 10.

Advantageously, the light source 824 is an infrared light emitting diode (LED). In a variant, the light source 824 is a LED of other wavelength, for example a diode emitting visible light or UV light. Alternatively any other suitable light source can be used, for example a laser diode.

Advantageously, the light detector 826 is an infrared phototransistor. In a variant, the light detector 826 is a photodiode or any other element for detecting the light intensity.

The light source 824 and the light detectors 826A and 826B are connected to a control unit 90, represented on figure 3 only, for the sake of simplicity, preferably implemented as an electronic component mounted on the printed circuit board 39. The electronic control unit 90 may be a processor, a microprocessor, a microcomputer, a logic gates system, a transistor or any other means suitable for performing calculations.

In a non-represented variant of the invention, the optical sensor 82 might include more than one light source 824 and/or a number light detector 826A, 826B different from two, preferably mounted on a single body 822.

An combination of the output signals S826A and S826B of the light detectors 826A and 826B forms an output signal S82 of the optical sensor 82 and is expressed as a voltage U provided to the control unit 90. This voltage U can take several values, depending on the intensity of a light beam emitted by the light source 824 and reaching the light detectors 826A and 826B. The measured intensity of the light beam corresponds to the quantity of light received by the light detector 826A and 826B.

The shuttle 84 extends along a longitudinal axis X84, parallel to axes X2, X14, X18 and X20 in mounted configuration of the auto-injector 2.

Advantageously, the longitudinal axis X84 is offset with respect to axes X2, X14, X18 and X20 in mounted configuration of the auto-injector 2. Advantageously, the longitudinal axis X84 is included in the median plane P82.

The shuttle 84 defines a volume V84 for the reception of the spring 16. This allows the spring 16 to exert a biasing effort on a disk-like portion 842 of the shuttle 84, this biasing effort being oriented parallel to the longitudinal axis X84, toward the needle cover 18, in the direction D2'.

The shuttle 84 also includes a longitudinal stem 844 configured to come into abutment against one of the lugs 188 of the needle cover 18. This allows transmitting to the needle cover 18 the biasing effort exerted by the spring 16 onto the shuttle 84, in the direction D2'.

On the other hand, the shuttle 84 includes a barrier 846 fixed, in translation around the longitudinal axis X84, with the longitudinal stem 844. The barrier 846 protrudes laterally with respect to the longitudinal axis X84, with respect to the stem 844.

Advantageously, the shuttle 84 is monobloc. In other words, parts 842, 844 and 846 are made in one piece.

The barrier 846 has a globally rectangular shape and is planar, with a thickness t846 compatible with its passage between the two legs of the body 822, more precisely between its light source 824 and its light detectors 826A and 826B. In other words, the barrier 846 is configured to slide along the longitudinal axis X84 and the directions D2 and D2', between the light source 824 and the light detectors 826A and 826B.

The barrier 846 is provided with three through-openings 848A, 848B and 848C, which follow each other, along the longitudinal axis X84. A first through-opening 848A is the furthest away from the longitudinal stem 844, a third through-opening 848C is the closest to the longitudinal stem 844 and a second through-opening 848B is intermediate, between through-openings 848A and 848C. 846A denotes a first transverse edge of the barrier 846, furthest away from the stem 844. 846B denotes a second transverse edge of the barrier 846, the closest to the stem 844. From edge 846A to edge 846B, the order of the through-openings is 848A, 848B, 848C.

The three through holes are identical. L848 denotes the length of a through-opening 848A, 848B or 848C measured parallel to the longitudinal axis X84.

846C denotes a portion of the barrier 846 located, along the longitudinal axis X84, between the first transverse edge 846A and the first through-opening 848A. 846D denotes a portion of the barrier 846 located, along the longitudinal axis X84, between the first through-opening 848A and the second through-opening 848B. 846E denotes a portion of the barrier 846 located, along the longitudinal axis X84, between the second through-opening 848B and the third through-opening 848C. 846F denotes a portion of the barrier 846 located, along the longitudinal axis X84, between the third through-opening 848C and the second transverse edge 846B.

The four portions 846C, 846D, 846E and 846F have the same dimensions. L846' denotes the length of a portion 846C, 846D, 846E or 846F measured parallel to the longitudinal axis X84.

826 denotes the set made of the first light detectors 826A and the second light detector 826B. L826 denotes the length of the set 826, measured parallel to the longitudinal axis X2.

The through-openings 848A, 848B and 848C increase the accuracy of the detection of the position of the shuttle 84 along the longitudinal axis X2, in particular when combined with use of more than one light detector, as in the first embodiment where light detectors 826A and 826B are used. It will be described in more details below.

The optical sensor 82 is configured to detect the position of the shuttle 84 along the longitudinal axis X2 of the auto-injector 2, this position being representative, firstly, of the presence or absence of the reusable module 50 and, secondly, of the longitudinal position of the needle cover 18, when the disposable module 50 is present.

On the other hand, the position of the shuttle 84 along the longitudinal axis X2 is detected via the position of its barrier 846.

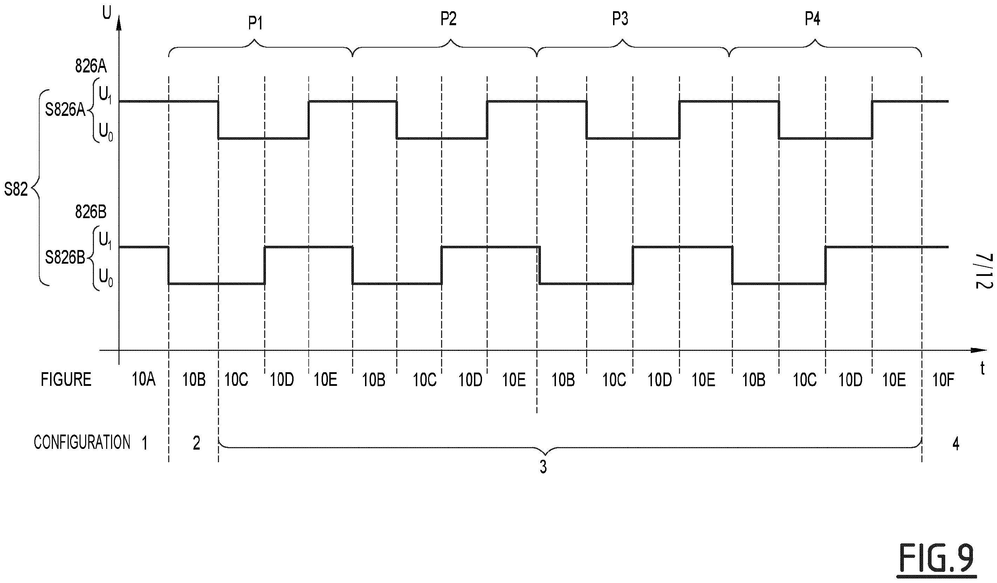

When the disposable module 50 is separated from the reusable module 30, the needle cover 18 does not limit the movement of the shuttle 84 along the longitudinal axes X2 and X84, in the direction D2', in which the shuttle 84 is biased by the spring 16. In this first configuration, represented on figure 4 and on figure 10A, the puller 8 along with the cap 10 are mounted on the body 6. Along the longitudinal axis X2, the barrier 846 is completely offset of the optical sensor 82. Therefore the barrier 846 does not interfere with the transmission of a light beam between the light source 824 and the light detectors 826A and 826B. In such a case, the voltage U of the output signal S82 of the optical sensor 82, which corresponds to the intensity of the light beam received by a respective light detector, takes its maximum value U1 for both light detectors 826A and 826B. This is represented as the configuration 1 in the graph presented on figure 9, while the relative positions of the barrier 846, through-openings 848A, 848B and 848C and light detectors 826A and 826B is shown as shown on figure 10A.

Then, if the disposable module 50 is mounted on the reusable module 30, the puller 8 and the stopper can be removed from the body 6, which makes the needle cover 18 exposed, since it protrudes out of the body 6 on the side of its second longitudinal end 64.

In figure 9, the configurations of the auto-injector 2 are identified by their order number on the lowest line of the figure, 1 for the first configuration, 2 for the second configuration, etc.

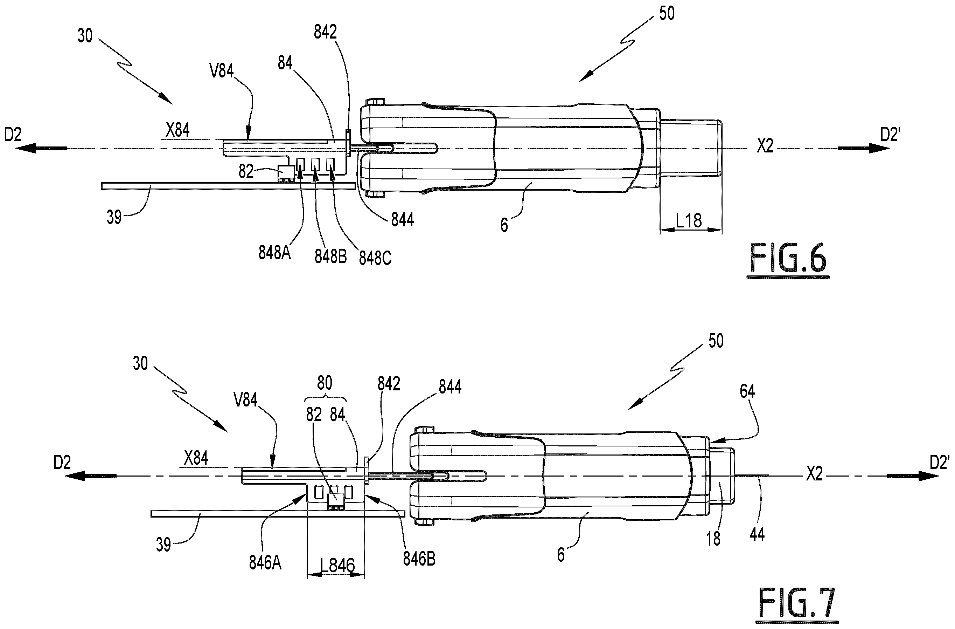

In the second configuration of figures 5 and 6, where the disposable module 50 is mounted on the reusable module 30, one of the lugs 188 of the needle cover 18 comes into abutment against the tip of the longitudinal stem 844 opposite to the disc-like portion 842. In this second configuration, the needle cover 18 and the shuttle 84 are coupled in translation along the longitudinal axis X2, under the biasing effort exerted by the spring 16. Abutment of the lug 188 pushes the shuttle 84 in the direction D2, along the longitudinal axis X2. This brings the shuttle 84 from the position of figure 4 and 10Bto the position of figure 5, where the barrier 846 partly interrupts the light beam transiting between the light source 824 and the light detectors 826A and 826B.

In the second configuration, the portion 846C of the barrier 846 is engaged into the body 822, between its two legs respectively supporting the light source 824 and the light detectors 826A and 826B. In this configuration, the second light detector 826B is hidden behind said portion 846C of the barrier 846, as it is shown on figure 10B, and the output signal S826B of the second light detector is at its minimum value U0. Preferably, the voltage U0 is equal to 0 Volts or close to this value. At the same time the output signal S826A the first light detector 826A does not change and stays at its maximum value U1. Thus, when this combination of the output signals S826A and S826B issued by two detectors appears in the output signal S82 during coupling of the modules 30 and 50, it means that said modules 30 and 50 have been correctly connected.

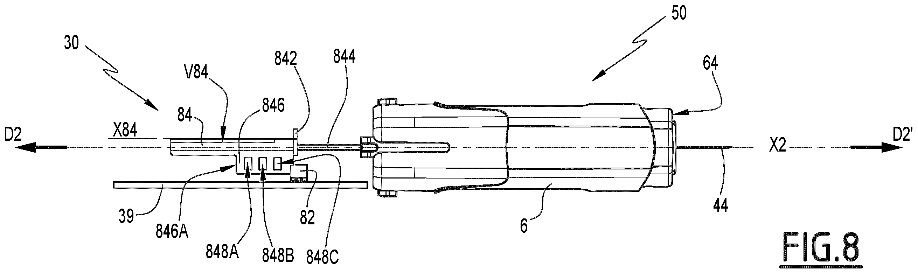

When the syringe 4 is used for injecting the content of the barrel 42, preferably into a human or animal body, the needle cover 18 moves from the second configuration of figures 5 and 6 through third configuration of figure 7 and then to the fourth configuration of figure 8.

The fourth configuration corresponds therefore to the fully retracted position of the needle cover 18. The third configuration of figure 7 corresponds to one of the intermediary positions of the needle cover 18 during its retraction. During use of the auto-injector 2, the needle cover 18 is pushed against the injection site, so that retraction of the needle cover 18 correspond to insertion of the needle 44 into the injection site, for example the patient's skin.

When the needle cover 18 is being retracted, the auto-injector 2 in a third configuration, where the needle cover pushes accordingly the stem 844 of the shuttle 84 with its lugs 188. Hence the stem 844, the barrier 846 and the through-openings 848A, 848B and 848 C together move between the light source 824 and the light detectors 826A and 826B. This causes sequential interrupting of the light path between the light source 824 and the light detectors 826A and 826B.

In this first, non-limiting embodiment, the length L848 of each through-opening 848A-848C and the length L846' of each portion 846C, 846D, 846E or 846F are both greater than the length L826 of the set 826 made of the two light detectors 826A and 826B. This results in that the light detectors 826A and 826B are sequentially covered by the barrier 846 when the needle cover retracts, as it is shown in figures 10C, 10D and 10E, which all correspond to the third configuration of the auto-injector 2.

During retraction of the needle cover 18, when the shuttle 84 further moves in the direction D2, the light detectors becomes both covered, as it is shown on figure 10C. In this position the output from both detectors is on the minimum level U0. This minimum value U0 is integrated in both output signals S826A and S826B, thus in the output signal S82 of the optical sensor.

A further movement of the needle cover 18 and shuttle 84 in the direction D2 causes that the through-opening 848A enters the detector area and that the light detector 826B becomes again uncovered. Hence the output from the light detector 826B is U1, while the output from the light detector 826A remains at the level U0. This position is shown on figure 10D. The values U0 are U1 are respectively integrated in the output signals S826A and S826B, thus in the output signal S82 of the optical sensor.

During further movement of the needle cover 18 and shuttle 84 in the direction D2, both detectors 826A and 826B are within the area of the through-opening 848A and therefore both detectors provide the output U1. This position is shown on figure 10e. The maximum value U1 is integrated in both output signals S826A and S826B, thus in the output signal S82 of the optical sensor.

Hence during transition of the barrier 846 and the through-opening 848A of the shuttle 84 in front of the set 826 made of the two detectors 826A and 826B, the following stages in the sequence of signals are obtained. The first stage is U1 on the light detector 826A and U0 on the light detector 826B, corresponding to the position of figure 10B. The second stage is U0 on both detectors 826A and 826B, corresponding to the position of figure 10C. The third stage is U0 on the light detector 826A and U1 one the light detector 826B, corresponding to the position of figure 10D. The fourth stage is U1 in both detectors 826A and 826B, corresponding to the position of figure 10E. The four stages are therefore determined by four possible combinations of the output signals S826A and S826B of the two light detectors, which may be in two different states. These four stages form together a first phase P1 of the retraction of the needle cover 18.

When the needle cover 18 is further retracted and moves the shuttle 84 in the direction D2, the portion 846D of the barrier 846 enters the detectors area, followed by the through-opening 848B. The sequence of signals for the portion 846D and the through-opening 848B transition is the same as the one explained for the positions of figures 10B to 10E and forms a second phase P2 shown in figure 9.

When the needle cover 18 is still further retracted and moves the shuttle 84 in the direction D2, the portion 846E of the barrier 846 enters the detectors area, followed by the through-opening 848C. The sequence of signals for the portion 846E and the through-opening 848B transition is the same as the one explained for the positions of figures 10B to 10E and forms a third phase P3 shown in figure 9.

The transition of the part 846F of the barrier 846 results in a fourth period P4 shown in figure 9. The sequence of signals for the portion 846F and the edge 846B transition is the same as the one explained for the positions of figures 10B to 10E.

Then, the position of figure 10F is reached, where the barrier 846 is completely offset with respect to the set of detectors 826. This corresponds to the fourth configuration of figure 8, mentioned here above.

In figure 10F, the optical sensor 82 is shifted to the right, with respect to figures 10A to 10E, in order to show the barrier 846 on its left.

As each of the length L848 of the openings 848A-848C is the same and the length L846' of the portions 846C to 846F, is the same, the signal sequences for each phase P1, P2, P3 and P4 are also the same, but for what concerns the position of figure 10F. The end of the period P4 corresponds to the fully retracted position of the needle cover 18.

As the signals S826A and S826B delivered by the two light detectors 826A and 826B are shifted in phase with respect to each other, the control unit 90 can distinguish between movement of the barrier 846 and therefore the needle cover 18 in both directions D2 and D2'. Hence the event of retraction interrupting and the movement of the needle cover 18 back to its extended position can be detected by the control unit 90. In this way, the full retraction of the needle cover is 18 can be detected as a unique event as it follows four subsequent phases P1-P2-P3-P4. If the needle cover 18 retraction is interrupted, for example during the P3 period and the needle cover 18 moves back to its initial, extended position, the optical sensor 82 detects that the movement corresponds to the sequence P1-P2-P3-P2-P1 rather than P1-P2-P3-P4.

The fully retracted position of the needle cover 18 means that the needle 44 is properly inserted and the injection can be initiated. This can be used for improvement of the operation of the auto-injector 2. For example, the injection can be prevented before reception of the signal from the control unit 90, said signal confirming that the needle cover 18 has been properly retracted. Alternatively or additionally, the control unit 90 may cause generation of the feedback signal for the user that the needle cover 18 is fully retracted and the injection can be started by pressing the button 38. Said feedback signal may be, for example, a sound signal, a visual signal, a tactile signal or their combination.

As a summary, when it moves from its extended position to its fully retracted position, the needle cover 18 translates along the direction D2 and pushes the shuttle 84, in particular its barrier 846 provided with the openings 848A, 848B and 848C, along the longitudinal axis X2, which changes the light intensity measured by the light detectors 826A and 826B in a specific sequence. This can be detected by the optical sensor 82 and such information can be further transmitted to the control unit 90.

Once injection has taken place, the needle cover 18 preferably reaches a position where it hides the needle 44 from the exterior of the auto-injector 2. This can be made under the action of the spring 16 pushing on the shuttle 84, which pushes on the needle cover 18 by the tip of its longitudinal stem 844, in the direction D2'. This brings the needle cover 18 back in a position where it can be, preferably, locked by the safety system 12. This position is advantageously the same as in the second configuration, so that the barrier 846 is located at the same position as in figures 5 and 6. During this movement of the needle cover 18 and the shuttle 84, the barrier 846 moves in front of the light detectors 826A and 826B in the direction D2'. Hence the optical sensor 82 detects the four periods presented on figure 9 in the reverse order, i.e. P4-P3-P2-P1.

An operation of the safety systems 12 usually depends only on the position of the needle cover 18, hence usually if the needle cover 18 has been properly retracted, it is locked in its fully extended position. The optical sensor 82 is able to detect the fully extended position the needle cover 18 of figures 5 and 6, when it follows the fully retracted position of the needle cover 18. Hence, optionally, the optical sensor 82 can also detect a locked state of the needle cover 18 and can, for example, provide the user with the feedback that the needle cover 18 is locked.

When, after use of the content of the syringe 4, the disposable module is detached from the reusable module 30, the shuttle 84 is urged by the spring 16 in the direction D2'. Then the shuttle 84 comes back to its initial position of the first configuration, where the barrier 846 is not located within the optical sensor 82, i.e. offset axially with respect to the set 826. When this movement follows the sequences corresponding to the full retraction of the needle cover 18 and its movement again to the extended position, the optical sensor 82 may optionally recognize this state as detachment of the modules 30 and 50.

In the first embodiment, the three openings 848A-848C provide four subsequent periods P1-P4 in the signal and the movement of the barrier 846 can be detected along with a direction of this movement. This embodiment can be modified by using a different number of openings in the barrier. The lower number of openings will make the barrier simpler, which can be particularly useful for the small devices. More openings will result in obtaining more than four periods in the signal, hence the axial position of the barrier along the axis X2 can be determined more precisely, if needed.

Therefore, the optical sensor 80 of the present invention is compatible with an axially movable needle cover 18 and capable of providing reliable information to the control unit 90. Such a control unit may be used to control, in particular, the actuation of the non-represented motor of the reusable module 30. The control unit may be used also for controlling operation of other electronic modules of the auto-injector 2, for example the modules for receiving and interpreting the user inputs provided by means of the button 38 and the slider 43 and for controlling the means for providing feedback to the user, for example light indicator 37.

L846 denotes the axial length of the barrier 846 measured parallel to the longitudinal axis X84, that is parallel to the longitudinal axis X2 when the shuttle 84 is mounted within the auto-injector 2.

L18 denotes the axial length of the stroke of the axial displacement of the needle cover 18 between its extended position represented on figures 5 and 6 and its fully retracted position represented on figure 8.

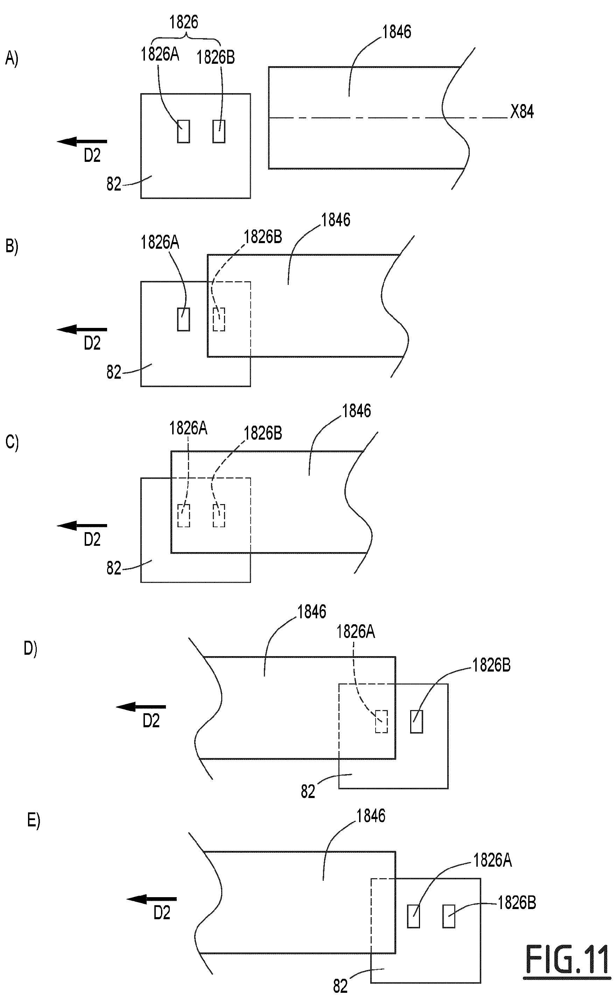

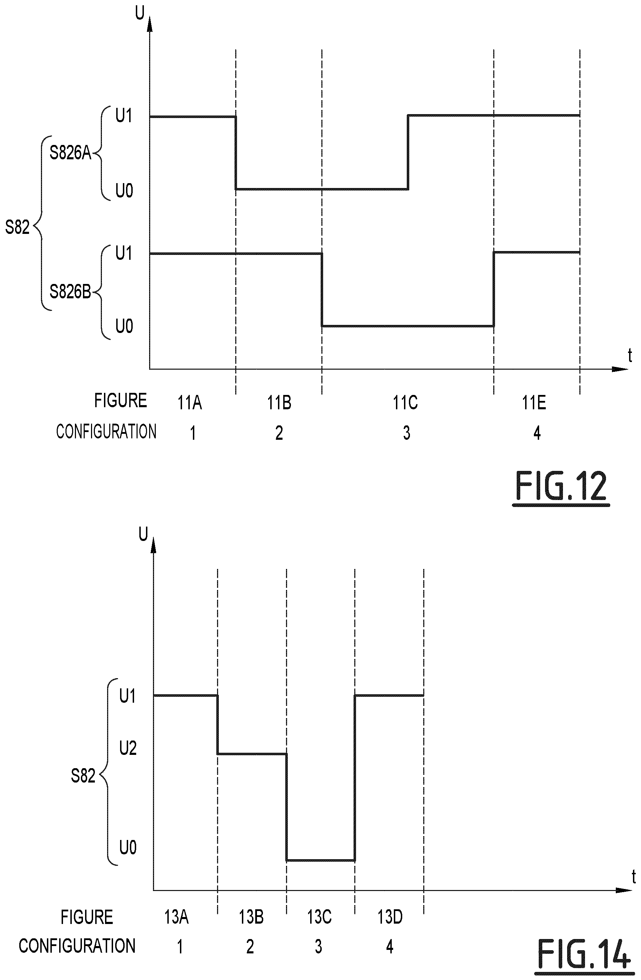

In the second embodiment of the invention represented on figures 11 and 12, the same elements as in the first embodiment have the same references plus 1000. If a reference is mentioned in the following description without being shown on figures 11 and 12 or shown on these figures without being mentioned in the description, it designates the same element as the one bearing the same reference in the first embodiment. Unless otherwise specified, the second embodiment works as the first embodiment. Hereafter, one describes mainly the differences between the first and second embodiments.

In the second embodiment of the invention, the barrier 1846 does not comprise openings. In other words, the barrier is solid. In this embodiment, two light detectors 1826A and 1826B form together a set of detectors 1826 and are used for detecting of the position of the barrier 1846 as it is shown in figures 11A-11E. The needle cover and other features of the auto-injector are, in particular, the same as for the first embodiment described above and they are not represented for simplicity.

The configuration of figure 11A corresponds to the state when the disposable and reusable modules are not connected to each other. In this configuration, the barrier 1846 of the shuttle is located outside the set of detectors 1826 and both detectors 1826A and 1826B give the output voltage at the maximum value U1. This state is marked on figure 12 as the first configuration and is detected as the state before connecting two modules.

When the disposable module and the reusable module are connected, the barrier 1846 of the shuttle is moved along the longitudinal axis X2, in the direction D2 and it enters the area of the set of detector 1826 covering the light detector 1826B, as shown in figure 11B. Hence, the output signal from this detector takes its minimum value U0, while the output signal from the light detector 1826A is still at its maximum value U1. This state is marked on figure 12 as the second configuration and, in this state, the optical system detects that two modules have been correctly connected.

When the needle cover is being retracted the barrier 1846 moves further in the direction D2. As a result, the barrier covers also the light detector 1826A, as shown on figure 11C. This results in that the output signal S1826A and S1826B of both detectors 826A and 826B is on its minimum value U0. Further displacement of the barrier 1846 in the direction D2 causes that the light detector 1826B becomes uncovered, as shown on figure 11d and it again gives an output value equal to its maximum, U1, while the light detector 1826B still gives output value U0. Both these positions of figures 11C and 11D are therefore related with the third configuration of figure 12 corresponding to the needle cover retraction.

When the needle cover is fully retracted, the barrier 1846 is on its extreme position towards the direction D2 and it does not cover any of the light detectors 1826A and 1826B, as shown on figure 11E. This corresponds to the fourth configuration marked on figure 12, in which both detectors 1826A and 1826B have again the output at value U1. In this way, the fully retracted position of the barrier 1846, and therefore of the needle cover, is detected as the fourth configuration following the sequences of signals of the previous configurations.

The other features and options of the first embodiment apply also to the second embodiment. In particular detecting of the fully retracted position of the needle cover being the confirmation that the needle is properly inserted may be indicated to the user by the feedback means and, alternatively or additionally, may result in allowing the mechanism to start the injection, which might have been prevented before proper needle cover retraction.

During displacement of the barrier 1846, the signals from the two sensors 1826A and 1826B are shifted in phase with respect to each other, hence the movement opposite to needle cover retraction can be detected. This allows detecting and interruption of the needle cover retraction and, if the needle cover has been properly retracted, movement of the needle cover back to its extended position and, preferably, locking of the needle cover by the safety system.

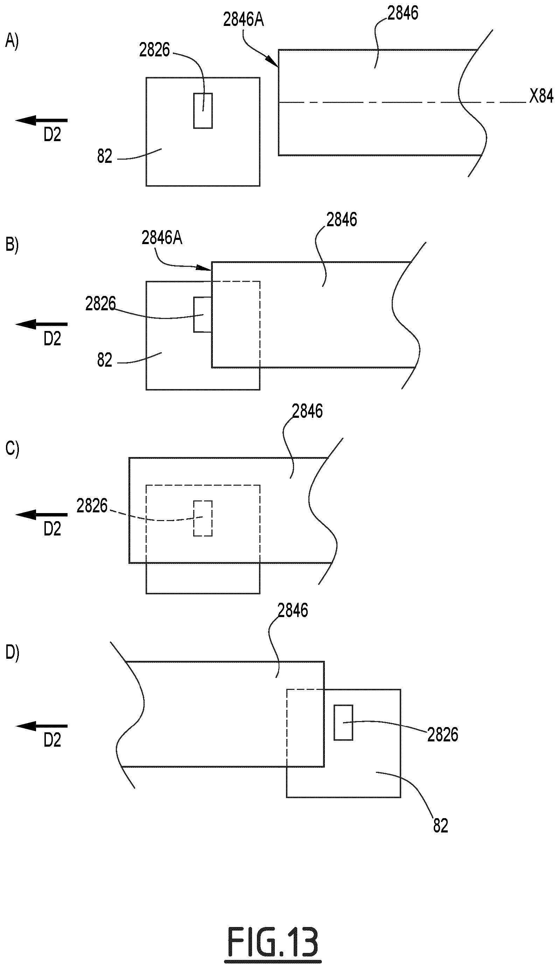

In the third embodiment of the invention represented on figures 13 and 14, the same elements as in the first embodiment have the same references plus 2000. If a reference is mentioned in the following description without being shown on figures 13 and 14 or shown on these figures without being mentioned in the description, it designates the same element as the one bearing the same reference in the first embodiment. Unless otherwise specified, the third embodiment works as the first embodiment. Hereafter, one describes mainly the differences between the first and second embodiments.

In the third embodiment of the invention, the barrier 2846 does not comprise openings. In other words, the barrier is solid. The optical sensor 82 comprises a single light detector 2826. The needle cover and other features of the auto-injector are, in particular, the same as for the first embodiment described above and they are not represented for simplicity.

The configuration of figure 13A, corresponds to the state when the disposable and reusable modules are not connected to each other. Then the barrier 2846 of the shuttle is located outside the light detector area and the light detector 2826 gives the output voltage at the maximum value U1. This state is marked on figure 14 as the first configuration and is detected as the state before connecting two modules.

When the disposable module and the reusable module are connected, the barrier 2846 of the shuttle is moved in the direction D2 and it enters the light detector area covering partially the light detector 2826 as it is shown in figure 13B. The output signal in this position is equal to U2 which is lower than U1. This state is marked on figure 14 as the second configuration and in this state the optical system detects that two modules have been correctly connected.

When the needle cover is being retracted the barrier 2846 moves further in the direction D2. As can be seen on figure 13C the barrier covers fully the light detector 2826 and its output is at the minimum value U0, where U0<U2<U1. This is related with the third configuration of figure 14 corresponding to the needle cover retraction.

In this third configuration, since the light detector 2826 is fully covered by the solid barrier 2846, light might not reach the light detector at all. Measurement of the light intensity gives a value equal to zero.

When the needle cover is fully retracted, the barrier 2846 is on its extreme position towards the direction D2 and it does not cover the light detector 2826, as shown on figure 13D. This corresponds to the fourth configuration marked on figure 14, in which the output signal from the light detector 2826 is again on the level U1.

In figures 12 and 14, the configurations of the auto-injector 2 are identified by their order number on the lowest line of the figure, 1 for the first configuration, 2 for the second configuration, etc.

The other features and options of the first embodiment apply also to the present, third embodiment. In particular detecting of the fully retracted position of the needle cover being the confirmation that the needle is properly inserted may be indicated to the user by the feedback means and, alternatively or additionally, may result in allowing the mechanism to start the injection, which might have been prevented before proper needle cover retraction.

In all embodiments, using an optical system 80 is advantageous since it allows detecting a position of the needle cover 18 without contact between the shuttle 84 and the optical sensor 82. Hence there is no additional friction generated in the device which would make its use more difficult. The optical sensors are also small and cheap elements which do not require sophisticated electronic components as they usually give the voltage values as the output. Also they do not require significant modification of the device as only simple mechanical barrier is sufficient for operation of the optical system.

In all embodiments, different wavelengths of the light can be used in the optical system 100 of invention. The infrared part of electromagnetic spectrum is particularly favorable due to its low sensitivity to ambient light, in particular a sensitivity lower than the visible part of the spectrum and UV light. Also infrared light does not have ionizing effect like UV. Hence, it is safer. In comparison with the optical sensor based on UV light, those utilizing infrared light are also cheaper.

When using the optical system 80 of the invention, the following steps are implemented:
a) emitting a light beam from the light source 824 toward the light detectors 826A and 826B, 1826A and 1826B or 2826;
b) measuring a light intensity via the light detectors 826A and 826B, 1826A and 1826B or 2826;
c) providing an output signal corresponding to the light intensity measured by the at least one light detector 826A, 826B, 1826A, 1826B or 2826;

As explained here above, this step b) allows generating the output signal S82 of the optical sensor 82 with one of the several values based on the intensity of light received at step b). These values may include either different voltage U0, U1 or U2 representative of the measure, or they may include various combinations of the voltage from two detectors, for example U0-U0, U0-U1, U1-U0, U1-U1. Other combinations, including more complex ones, can be also used and are within the scope of the current invention. For example there may be three detectors, for which the measured voltage may be at the level of U1 or U0 resulting in 8 combinations of these values which may be used for defining various configurations.

Considering that the above-mentioned values of voltage of one light detector or combination of voltage measurements from two light detectors are representative of the intensity of light received at step b), it is further possible to compare said values of the output signal S82, provided by the optical sensor 82 to the control unit 90, to a threshold values or to known combinations of the threshold values. This occurs in a further comparison step d) of the method of the invention.

In a further step e), it is then possible, based on the comparison of step d), to determine a state of the light detectors 826A and 826B, 1826A and 1826B or 2826, then to assess the position of the needle cover 18 in a last step f), based on the position of the barrier 846 and of the shuttle 84 detected via the optical sensor 82.

In the first and second embodiments, an assumption is made that the state of the two detectors 846A and 846B or 1846A and 1846B can take one of values U1 and U0, and that each value corresponds to one of the following four configurations are identified,
i) no disposable module 50 is mounted on the reusable module 30;
ii) the disposable module 50 is mounted on the reusable module 30 and the needle cover 18 is in its extended position;
iii) the disposable module 50 is mounted on the reusable module 30 and the needle cover 18 moves from its extended position to its retracted position;
iv) the disposable module 50 is mounted on the reusable module 30 and the needle cover 18 is in its retracted position,
wherein configuration i) corresponds to a state when the two detectors 846A and 846B, or 1846A and 1846B take the first value U1, configuration ii) corresponds to a state when at least one detector 846A, 846B, 1846A and 1846B takes the minimum value U0 and configuration iv) corresponds to a state when the two detectors 846A and 846B, or 1846A and 1846B take the first value U1, following sequential changes in the state of the two detectors 846A, 846B, 1846A and 1846B starting from the configuration ii)

In the third embodiment, an assumption is made that the state of the light detector 2846 can take one of a first value U1, a second value U2 and a third value U3 and that each value corresponds to one of the following four configurations:
i) no disposable module 50 is mounted on the reusable module 30;
ii) the disposable module 50 is mounted on the reusable module 30 and the needle cover 18 is in its extended position;
iii) the disposable module 50 is mounted on the reusable module 30 and the needle cover 18 moves from its extended position to its retracted position;
iv) the disposable module 50 is mounted on the reusable module 30 and the needle cover 18 is in its retracted position,
wherein the state is the same for the configuration i) and the configuration iv).

In the embodiment represented on the figures, the needle cover 18 and the shuttle 84 are two separate elements coupled with each other under the elastic biasing effort exerted by the spring 16. In a non-represented alternative embodiment of the invention, the shuttle 84 is in one part with the needle cover 18.

In the example of the figures, the shuttle 84 is monobloc. In particular, the barrier 846 is in one piece with the longitudinal stem 844. In a non-represented variant of the invention, the shuttle 84 can be made of several parts assembled together.

The embodiments and variants of the invention mentioned here above may be combined, in any technically feasible way, in order to generate new embodiments of the invention, in the framework of the appended set of claims.

## Claims

1. An optical system (80) for detecting a position of a needle cover (18) in an auto-injector (2), said needle cover being able to translate between a retracted position and an extended position, the optical system comprising
- at least one light source (824);
- at least one light detector (826A, 826B, 1826A, 1826B, 2826); and
- a barrier (846, 1846, 2846) able to move in front of the light detector,
wherein
- a movement of the needle cover (18) along a longitudinal axis (X2) causes an axial displacement of the barrier (846, 1846, 2846) and changes a light intensity measured by the light detector (826A, 826B, 1826A, 1826B, 2826), and
- the optical system (80) is configured to detect an axially retracted position of the needle cover (18) due to a variation of the light intensity measured by the light detector (826A, 826B, 1826A, 1826B, 2826).

2. The optical system of claim 1, comprising at least two light detectors (826A, 826B, 1826A, 1826B).

3. The optical system of claim 2, wherein during a movement of the needle cover (18) along the longitudinal axis (X2), output signals of the at least two light detectors (826A, 826B, 1826A, 1826B) are shifted in phase.

4. The optical sensor of any preceding claim, wherein the barrier (846) comprises at least one opening (848A, 848B, 848C).

5. The optical system of claim 4, comprising at least two light detectors (826A, 826B)

6. The optical system of claim 5, wherein, during a movement of the needle cover (18) along the longitudinal axis (X2), output signals (S826A, S826B) of the at least two light detectors (826A, 826B) change periodically.

7. An auto-injector (2) for use with a syringe (4) comprising an injection needle (44), the auto-injector comprising a needle cover (18) for covering the injection needle, the needle cover being mobile in translation along an axial direction (D2, D2'), between an extended position and a retracted position, wherein the auto-injector comprises an optical system (80) according to any preceding claim.

8. The auto-injector of claim 7 comprising a reusable module (30) and an disposable module (50) including the needle cover (18), wherein mounting of the disposable module (50) on the reusable module (30) causes an axial movement of the barrier (846).

9. The auto-injector of claim 8, wherein the optical system (80) is configured to identify a correct mounting of the disposable module (50) on the reusable module (30).

10. The auto-injector of one of claims 8 and 9, wherein the optical system (80) is configured to detect that the needle cover (18) is in its extended position after being previously in the retracted position.

11. The auto-injector of claim 7, comprising a reusable module (30) and an disposable module (50) including the needle cover (18), wherein
- the light source (824) and the at least one light detector (826A, 826B, 1826A, 1826B, 2826) of the optical system (80) belong to the reusable module (30),
- the barrier (846) allows the same amount of light to reach the at least one light detector (826A, 826B, 1826A, 1826B, 2826)
• when the disposable module (50) is mounted on the reusable module (30) and the needle cover (18) is in its retracted position and
• when the disposable module (50) is not mounted on the reusable module (30).

12. A method for detecting a position of a needle cover (18) in an auto-injector (2) according to one of claims 7 to 11, comprising :
a) emitting a light beam from the light source (824) toward the at least one light detector (826A, 826B, 1826A, 1826B, 2826);
b) measuring a light intensity via at least one light detector (826A, 826B, 1826A, 1826B, 2826);
c) providing an output signal corresponding to the light intensity measured by the at least one light detector (826A, 826B, 1826A, 1826B, 2826);
d) comparing the output signal of step c) to at least one known threshold value;
e) determining a state of the at least one light detector, based on the comparison of step d); and
f) determining the position of the needle cover (18).

13. The method of claim 12 implemented with an auto-injector (2) according to claim 8, wherein, in step e), an assumption is made that the state of the light detector (2846) can take one of a first value (U1), a second value (U2) and a third value (U3) and that each value corresponds to one of the following four configurations:
i) no disposable module (50) is mounted on the reusable module (30);
ii) the disposable module (50) is mounted on the reusable module (30) and the needle cover (18) is in its extended position;
iii) the disposable module (50) is mounted on the reusable module (30) and the needle cover (18) moves from its extended position to its retracted position;
iv) the disposable module (50) is mounted on the reusable module (30) and the needle cover (18) is in its retracted position,
wherein the state is the same for the configuration i) and the configuration iv).

14. The method of claim 12 implemented with an auto-injector (2) according to claim 8, wherein, in step e), an assumption is made that the state of the two detectors (846A, 846B, 1846A, 1846B) can take one of a first value (U1), and a minimum value (U0), and that each value corresponds to one of the following four configurations are identified,
i) no disposable module (50) is mounted on the reusable module (30);
ii) the disposable module (50) is mounted on the reusable module (30) and the needle cover (18) is in its extended position;
iii) the disposable module (50) is mounted on the reusable module (30) and the needle cover (18) moves from its extended position to its retracted position;
iv) the disposable module (50) is mounted on the reusable module (30) and the needle cover (18) is in its retracted position,
wherein configuration i) corresponds to a state when the two detectors (846A, 846B, 1846A, 1846B) take the first value (U1), configuration ii) corresponds to a state when at least one detector (846A, 846B, 1846A, 1846B) takes the minimum value (U0) and configuration iv) corresponds to a state when the two detectors (846A, 846B, 1846A, 1846B) take the first value (U1), following sequential changes in the state of the two detectors (846A, 846B, 1846A, 1846B) starting from the configuration ii) 15.- The method of any of claims 12-14, wherein the light beam is a beam of an infrared light.
